# EUROPÄISCHE PATENTSCHRIFT

(11) **EP 0 741 690 B1**
(45) Veröffentlichungstag und Bekanntmachung des Hinweises auf die Patenterteilung: **28.10.1998**
(21) Anmeldenummer: 95905639.1
(22) Anmeldetag: 20.01.1995
(51) Int. Cl.: C07C 213/10, C07C 209/84, C07C 227/40, C07C 231/24

(54) **VERFAHREN ZUR VERMINDERUNG DES RESTGEHALTES AN FREIEM ALKYLIERUNGSMITTEL IN WÄSSRIGEN LÖSUNGEN STICKSTOFFHALTIGER TENSIDE**
METHOD OF REDUCING THE RESIDUAL CONTENT OF FREE ALKYLATION AGENT IN AQUEOUS SOLUTIONS OF NITROGEN-CONTAINING SURFACTANTS
PROCEDE DE REDUCTION DE LA TENEUR RESIDUELLE EN AGENTS ALKYLANTS LIBRES DANS DES SOLUTIONS AQUEUSES DE TENSIOACTIFS CONTENANT DE L'AZOTE

(30) Priorität: 29.01.1994 DE 4402695
(43) Veröffentlichungstag der Anmeldung: 13.11.1996
(73) Patentinhaber: Henkel Kommanditgesellschaft auf Aktien, 40191 Düsseldorf (DE)
(72) Erfinder: BEHLER, Ansgar, D-46240 Bottrop (DE); FABRY, Bernd, D-41352 Korschenbroich (DE); PI, Rafael, E-08400 Granollers (ES); BIGORRA, Joaquim, E-08023 Sabadell (ES); PONSATI, Oriol, E-08025 Barcelona (ES)
(86) Internationale Anmeldenummer: EP9500210
(87) Internationale Veröffentlichungsnummer: WO9520566

(56) Entgegenhaltungen:
- WO-A-91/08193
- WO-A-92/03406

## Beschreibung

Die Erfindung betrifft ein Verfahren zur Verminderung des Restgehaltes an freiem Alkylierungsmittel in wäßrigen Lösungen stickstoffhaltiger Tenside, bei dem man Lösungen von kationischen, amphoteren und/oder zwitterionischen Tensiden mit Alkanolaminen nachbehandelt.

### Stand der Technik

Kationische Tenside, beispielsweise vom Typ der Esterquats oder quartären Ammoniumverbindungen stellen wichtige Produkte zur Herstellung von Wäscheweichspülern, Avivagemitteln für Haarbehandlungszwecke, Antistatika und Desinfektionsmitteln dar. Amphotere bzw. zwitterionische Tenside zeichnen sich durch ausgezeichnete Reinigungs- und Schaumeigenschaften aus und eignen sich als Co-Tenside für eine Vielzahl von oberflächenaktiven Mittel wie z.B. milde Haarshampoos. Die drei Tensidtypen weisen als verbindendes Strukturelement ein quartäres Stickstoffatom auf. Zu ihrer Herstellung geht man in jedem Fall von Aminen oder Fettsäureamidoaminen aus, die mit einem Alkylierungsmittel, beispielsweise Methylchlorid, Benzylchlorid oder Dimethylsulfat umgesetzt werden.

Da die Alkylierung nicht quantitativ erfolgt, weisen die quaternierten Stickstoffverbindungen ohne Nachbehandlung einen Restgehalt von 0,1 bis 3 Gew.-% des Alkylierungsmittels auf. Da es zur Erzielung toxikologisch unbedenklicher Produkte erforderlich ist, den Gehalt an Methylchlorid, Benzylchlorid oder Dimethylsulfat in den Produkten auf ein möglichst niedriges Niveau herabzusenken, hat es in der Vergangenheit nicht an Versuchen gemangelt, dieses Ziel durch eine geeignete Aufarbeitung sicherzustellen. Üblicherweise wird dabei der Gehalt an freiem Alkylierungsmittel durch mehrstündige thermische Behandlung bis auf einen geringen Betrag herabgesetzt. Diese Vorgehensweise ist jedoch mit einem hohen Aufwand an Zeit und Energie verbunden.

In **J.Falbe (ed.), "Surfactants in consumer products", Springer Verlag, Berlin, 1987, S.107-114** wird vorgeschlagen, den Restgehalt an freiem Alkylierungsmittel durch eine Nachbehandlung mit Ammoniak zu vermindern. Gemäß der Lehre der Japanischen Offenlegungsschrift **JP-A 60/178846** lassen sich aromatische quartäre Ammoniumverbindungen durch Umsetzung von aromatischen Aminen mit Benzylchlorid herstellen, wobei überschüssiges Alkylierungsmittel nach der Reaktion durch Zugabe von linearen Aminen abgefangen wird. Zur Verminderung des freien Gehaltes an Alkylierungsmittel wird schließlich in den Deutschen Offenlegungsschriften **DE-A1 3939264** und **DE-A1 4026184** (Henkel) vorgeschlagen, die wäßrigen Tensidlösungen mit tertiären Aminen, Aminosäuren oder Oligopeptiden zu behandeln. Es hat sich jedoch gezeigt, daß diese Verfahren zur Bildung anderer, ebenfalls unerwünschter Zwischenprodukte führen können und/oder nicht mit zufriedenstellender Geschwindigkeit ablaufen.

Die Aufgabe der Erfindung bestand somit darin, ein neues Verfahren zur Nachbehandlung von stickstoffhaltigen Tensiden zu entwickeln, das eine deutliche Verminderung des Gehaltes an freiem Alkylierungsmittel sicherstellt und frei von den geschilderten Nachteilen ist.

### Beschreibung der Erfindung

Gegenstand der Erfindung ist ein Verfahren zur Verminderung des Restgehaltes an freiem Alkylierungsmittel in wäßrigen Lösungen stickstoffhaltiger Tenside bei dem man Lösungen kationischer, amphoterer und/oder zwitterionischer Tenside mit Monoethanolamin bei erhöhter Temperatur und gegebenenfalls erhöhtem Druck nachbehandelt.

Überraschenderweise wurde gefunden, daß diese Art der Aufarbeitung gegenüber den bekannten Verfahren des Stands der Technik in deutlich kürzeren Zeiten zu niedrigeren Restgehalten an freiem Alkylierungsmittel in den Tensidlösungen führt. Die Erfindung beruht auf der Erkenntnis, daß die Alkanolamine in den wäßrigen Tensidlösungen sehr gut löslich sind und bei erhöhter Temperatur rasch und vollständig mit den Spuren nicht umgesetzten Alkylierungsmittels abreagieren.

### Esterquats

Unter der Bezeichnung Esterquats werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Stellvertretend für den umfangreichen Stand der Technik sei an dieser Stelle auf die Druckschriften **US 3,915,867, US 4,370,272, EP-A2 0239910, EP-A2 0293955, EP-A2 0295739** und **EP-A2 0309052** verwiesen.

Die quaternierten Fettsäuretriethanolaminestersalze folgen der Formel **(I)** 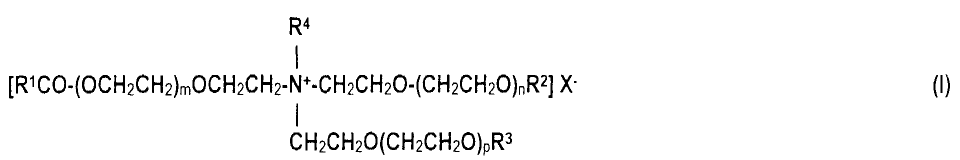 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H- Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt.

Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg- bzw. Palmfettsäure (lodzahl 0 bis 40) ab. Aus anwendungstechnischer Sicht haben sich quaternierte Fettsäuretriethanolaminestersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht. Neben den quaternierten Fettsäuretriethanolaminestersalzen kommen als Esterquats ferner auch quaternierte Estersalze von Fettsäuren mit Diethanolalkylaminen der Formel **(II)** in Betracht, 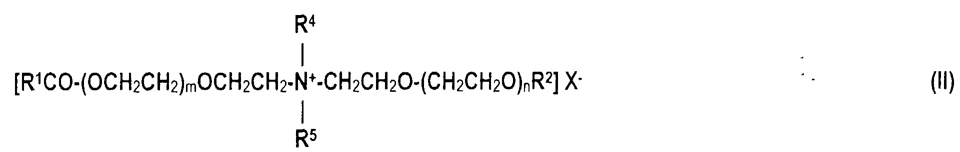 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Als weitere Gruppe geeigneter Esterquats sind schließlich die quaternierten Estersalze von Fettsäuren mit 1,2-Dihydroxypropyldialkylaminen der Formel **(III)** zu nennen, 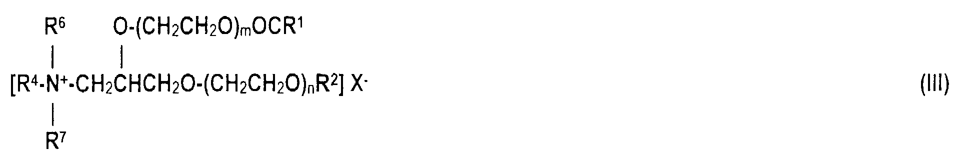 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für (I) genannten Beispiele auch für die Esterquats der Formeln **(II)** und **(III)**. Üblicherweise gelangen die Esterquats in Form 50 bis 90 Gew.-%iger alkoholischer Lösungen in den Handel, die bei Bedarf problemlos mit Wasser verdünnt werden können.

### Quartäre Ammoniumverbindungen

Die im Sinne des erfindungsgemäßen Verfahrens als kationische Tenside in Frage kommenden quartären Ammoniumverbindungen (QAV) folgen der Formel **(IV)**, 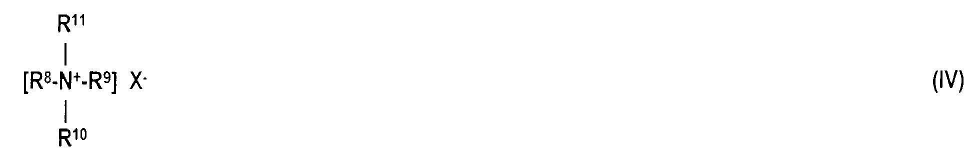 in der R⁸ und R⁹ unabhängig voneinander für gegebenenfalls hydroxysubstituierte Alkylreste mit 6 bis 22 Kohlenstoffatomen, R¹⁰ für gegebenenfalls hydroxysubstituierte Alkylreste mit 1 bis 22 Kohlenstoffatomen, R¹¹ für Alkylreste mit 1 bis 4 Kohlenstoffatomen und X für Halogenid, Alkylsulfat oder Alkylphosphat steht. Typische Beispiele sind vor allem solche QAV, die über zwei lange und zwei kurze Alkylreste als Substituenten verfügen und ihrer Struktur nach als Rohstoffe für die Herstellung von Faser-, Textil- oder Haaravivagemittel geeignet sind. Ein typisches Beispiel für diese Gruppe von Verbindungen ist das Dimethyldistearylammoniumchlorid (DMDSAC).

### Amphotere bzw. zwitterionische Tenside

Im Sinne des erfindungsgemäßen Verfahrens kommen als amphotere bzw. zwitterionische Tenside beispielsweise Alkylbetaine der Formel **(V)** in Frage, 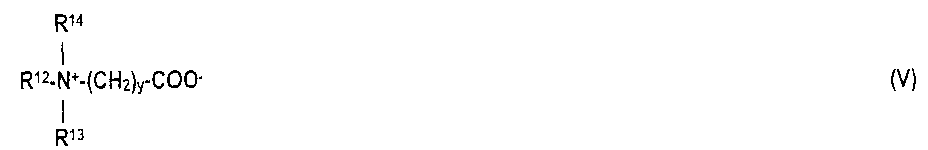 in der R¹² und R¹³ unabhängig voneinander für gegebenenfalls hydroxysubstituierte Alkylreste mit 6 bis 22 Kohlenstoffatomen, R¹⁴ für Wasserstoff oder eine Methylgruppe und y für 1, 2 oder 3 steht. Ebenfalls als amphotere bzw. zwitterionische Tenside geeignet sind Alkylamidobetaine der Formel **(VI)**, 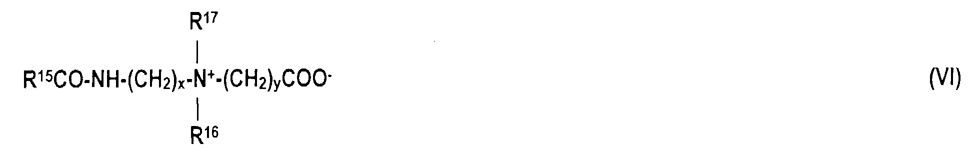 in der R¹⁵CO für einen aliphatischen Acylrest mit 6 bis 22 Kohlenstoffatomen, R¹⁶ für einen gegebenenfalls hydroxysubstituierten Alkylrest mit 1 bis 22 Kohlenstoffatomen, R¹⁷ für Wasserstoff oder eine Methylgruppe und x und y unabhängig voneinander für 1, 2 oder 3 steht.

### Nachbehandlung

Die Nachbehandlung der Tensidlösungen erfolgt üblicherweise, indem man die Lösungen mit dem Nachbehandlungsmittel über einen Zeitraum von 1 bis 4, vorzugsweise 1 bis 2 h, bei einem pH-Wert von 7,5 bis 9,5, der sich automatisch einstellt, und einer Temperatur von 60 bis 120, vorzugsweise 70 bis 90°C erwärmt. Allgemein gilt, daß die Senkung des Restgehaltes an Alkylierungsmittel um so rascher erfolgt, je höher die Temperatur bei der Nachbehandlung ist. Die Nachbehandlung erfolgt in der Regel bei Normaldruck. Zur Beschleunigung der Reaktion ist es jedoch möglich, bei erhöhtem Druck, beispielsweise von 1 bis 2 bar, und einer Temperatur von bis zu 120°C im Druckautoklaven zu arbeiten. Durch die Nachbehandlung wird der Restgehalt an freiem Alkylierungsmittel, insbesondere Methylchlorid und Dimethylsulfat innerhalb von 1 bis 2 h auf Werte unterhalb 5, vorzugsweise unter 1 ppm - bezogen auf den Feststoffgehalt der Tensidlösungen - herabgesetzt.

### Gewerbliche Anwendbarkeit

Die nach dem erfindungsgemäßen Verfahren erhältlichen wäßrigen Lösungen kationischer, amphoterer und/oder zwitterionischer Tenside sind bis in den Nachweisbereich frei von Spuren freien Alkylierungsmittels und eignen sich daher zur Herstellung von Mitteln zur Faser-, Textil- und Haaravivage.

### Beispiele

**Ausgangsstoffe**. Bei den eingesetzten Ausgangsstoffen handelt es sich ohne Ausnahme um Labormuster, in denen zum Nachweis der Wirksamkeit des erfindungsgemäßen Verfahrens durch künstliches Aufstocken ein Gehalt an freiem Alkylierungsmittel von 1000 ppm eingestellt worden war.
(A) Methylquaternierter Fettsäuretriethanolaminester in Form des Methylsulfatsalzes;
(B) Dimethyldistearylammoniumchlorid;
(C) Betain auf Basis Kokosfettsäuredimethylaminopropylamid

**Durchführung der Versuche**. In einem 1-1-Dreihalskolben mit Rührer, Rückflußkühler und Innenthermometer wurden jeweils 500 g der Tenside A, B und C in Form 10 Gew.-%iger wäßriger Lösungen vorgelegt und mit 0,5 g, entsprechend 1 Gew.-% - bezogen auf den Feststoffgehalt der Tensidlösungen - der Nachbehandlungsmittel versetzt. Die Reaktionsmischungen wurden bei pH = 8 bis 8,5 und einer Temperatur von T = 90°C über einen Zeitraum von t = 1 bis 2 h erhitzt und anschließend auf Raumtemperatur abgekühlt. Der Restgehalt an freiem Alkylierungsmittel wurde gaschromatographisch ermittelt. Die Ergebnisse sind in Tabelle 1 zusammengefaßt.

**Tabelle 1**

| **Nachbehandlung von wäßrigen Tensidlösungen** | | | | | | |
|---|---|---|---|---|---|---|
| | | | | **Alkylierungsmittel** | | |
| **Bsp.** | **Tensid** | **Nachbehandlungsmittel** | **t h** | **Typ** | **vorher ppm** | **nachher ppm** |
| 1 | A | Monoethanolamin | 1 | DMS | 1000 | 3 |
| 2 | A | Monoethanolamin | 2 | DMS | 1000 | < 1 |
| 3 | B | Monoethanolamin | 1 | MeCl | 1000 | 2 |
| 4 | B | Monoethanolamin | 2 | MeCl | 1000 | < 1 |
| 5 | C | Monoethanolamin | 1 | MCE | 1000 | 15 |
| 6 | C | Monoethanolamin | 2 | MCE | 1000 | < 1 |
| V1 | A | Ammoniak | 1 | DMS | 1000 | 114 |
| V2 | A | Methylamin | 1 | DMS | 1000 | 79 |
| V3 | A | Glycin | 1 | DMS | 1000 | 8 |
| t= Nachbehandlungszeit, DMS = Dimethylsulfat, MeCl = Methylchlorid, MCE = Monochloressigsäure | | | | | | |

## Patentansprüche

1. Verfahren zur Verminderung des Restgehaltes an freiem Alkylierungsmittel in wäßrigen Lösungen stickstoffhaltiger Tenside, bei dem man Lösungen kationischer, amphoterer und/oder zwitterionischer Tenside mit Monoethanolamin bei erhöhter Temperatur und gegebenenfalls erhöhtem Druck nachbehandelt.

2. Verfahren nach Anspruch 1, **dadurch gekennzeichnet**, daß man als kationische Tenside Esterquats der Formel (I) einsetzt, 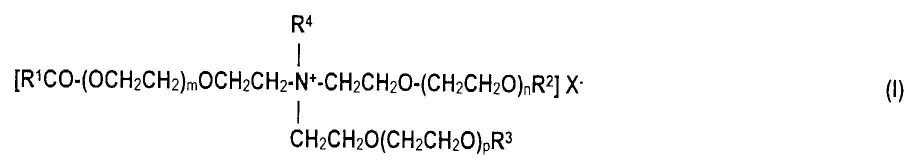 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Verfahren nach Anspruch 1, **dadurch gekennzeichnet**, daß man als kationische Tenside Esterquats der Formel (II) einsetzt, 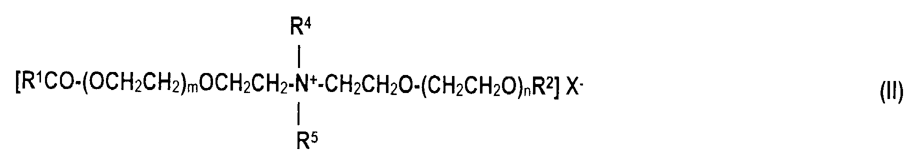 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Verfahren nach Anspruch 1, **dadurch gekennzeichnet**, daß man als kationische Tenside Esterquats der Formel **(III)** einsetzt, 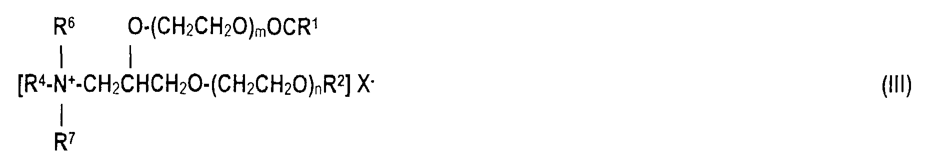 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verfahren nach Anspruch 1, **dadurch gekennzeichnet**, daß man als kationische Tenside quartäre Ammoniumverbindungen der Formel **(IV)** einsetzt, 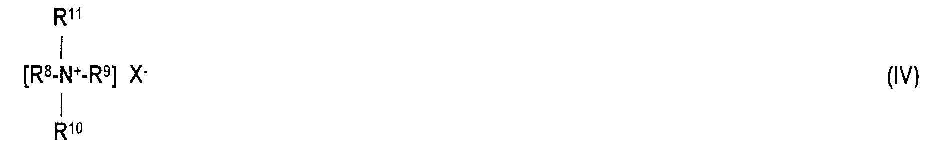 in der R⁸ und R⁹ unabhängig voneinander für gegebenenfalls hydroxysubstituierte Alkylreste mit 6 bis 22 Kohlenstoffatomen, R¹⁰ für gegebenenfalls hydroxysubstituierte Alkylreste mit 1 bis 22 Kohlenstoffatomen, R¹¹ für Alkylreste mit 1 bis 4 Kohlenstoffatomen und X für Halogenid, Alkylsulfat oder Alkylphosphat steht.

6. Verfahren nach Anspruch 1, **dadurch gekennzeichnet**, daß man als amphotere bzw. zwitterionische Tenside Alkylbetaine der Formel **(V)** einsetzt, 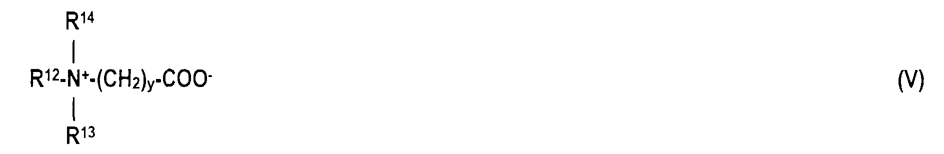 in der R¹² und R¹³ unabhängig voneinander für gegebenenfalls hydroxysubstituierte Alkylreste mit 6 bis 22 Kohlenstoffatomen, R¹⁴ für Wasserstoff oder eine Methylgruppe und y für 1, 2 oder 3 steht.

7. Verfahren nach Anspruch 1, **dadurch gekennzeichnet**, daß man als amphotere bzw. zwitterionische Tenside Alkylamidobetaine der Formel **(VI)** einsetzt, 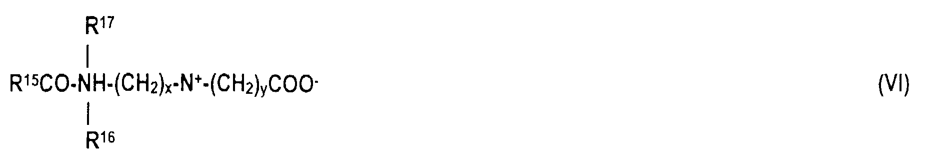 in der R¹⁵CO für einen aliphatischen Acylrest mit 6 bis 22 Kohlenstoffatomen, R¹⁶ für einen gegebenenfalls hydroxysubstituierten Alkylrest mit 1 bis 22 Kohlenstoffatomen, R¹⁷ für Wasserstoff oder eine Methylgruppe und x und y unabhängig voneinander für 1, 2 oder 3 steht.

8. Verfahren nach den Ansprüchen 1 bis 7, **dadurch gekennzeichnet**, daß man die Lösungen mit 0,1 bis 10 Gew.-% - bezogen auf den Feststoffgehalt - Monoethanolamin nachbehandelt.

9. Verfahren nach den Ansprüchen 1 bis 9, **dadurch gekennzeichnet**, daß man die Lösungen über einen Zeitraum von 1 bis 4 h, bei einem pH-Wert von 7,5 bis 9,5 und einer Temperatur von 60 bis 120°C nachbehandelt.

## Claims

1. A process for reducing the residual content of free alkylating agent in aqueous solutions of nitrogen-containing surfactants, in which solutions of cationic, amphoteric and/or zwitterionic surfactants are aftertreated with monoethanolamine at elevated temperature and optionally elevated pressure.

2. A process as claimed in claim 1, characterized in that esterquats corresponding to formula (I): 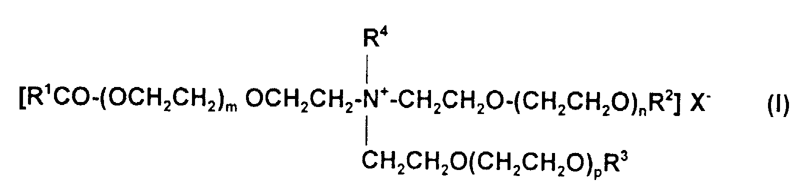 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate, are used as the cationic surfactants.

3. A process as claimed in claim 1, characterized in that esterquats corresponding to formula (II): 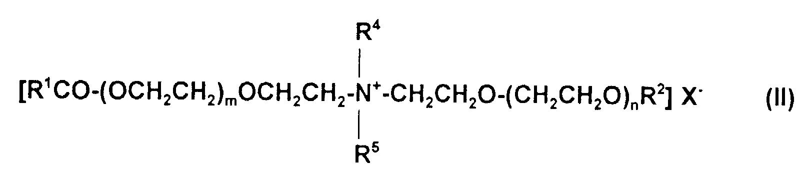 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X is halide, alkyl sulfate or alkyl phosphate,
are used as the cationic surfactants.

4. A process as claimed in claim 1, characterized in that esterquats corresponding to formula (III): 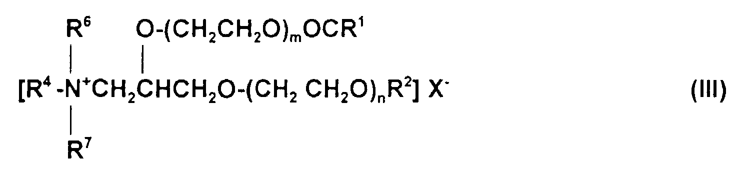 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X is halide, alkyl sulfate or alkyl phosphate,
are used as the cationic surfactants.

5. A process as claimed in claim 1, characterized in that quaternary ammonium compounds corresponding to formula (IV): 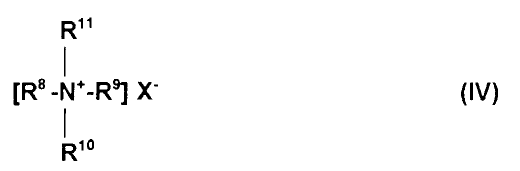 in which R⁸ and R⁹ independently of one another represent optionally hydroxysubstituted alkyl groups containing 6 to 22 carbon atoms, R¹⁰ represents optionally hydroxysubstituted alkyl groups containing 1 to 22 carbon atoms, R¹¹ represents alkyl groups containing 1 to 4 carbon atoms and X is halide, alkyl sulfate or alkyl phosphate
are used as the cationic surfactants.

6. A process as claimed in claim 1, characterized in that alkylbetaines corresponding to formula (V): 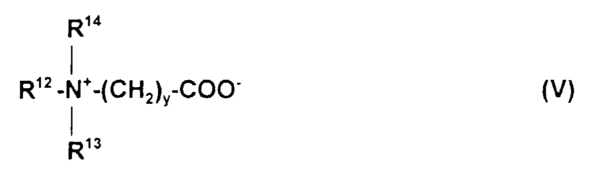 in which R¹² and R¹³ independently of one another represent optionally hydroxysubstituted alkyl groups containing 6 to 22 carbon atoms, R¹⁴ is hydrogen or a methyl group and y = 1, 2 or 3,
are used as the amphoteric or zwitterionic surfactants.

7. A process as claimed in claim 1, characterized in that alkyl amidobetaines corresponding to formula (VI): 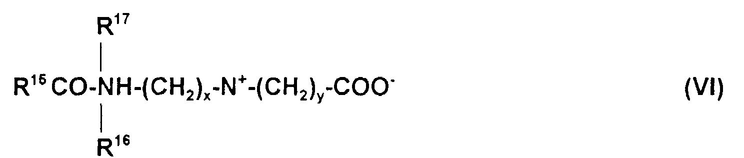 in which R¹⁵CO is an aliphatic acyl group containing 6 to 22 carbon atoms, R¹⁶ is an optionally hydroxysubstituted alkyl group containing 1 to 22 carbon atoms, R¹⁷ is hydrogen or a methyl group and x and y independently of one another have values of 1, 2 or 3,
are used as the amphoteric or zwitterionic surfactants.

8. A process as claimed in claims 1 to 7, characterized in that the solutions are aftertreated with 0.1 to 10% by weight, based on their solids content, of monoethanolamine.

9. A process as claimed in claims 1 to 9, characterized in that the solutions are aftertreated for 1 to 4 h at a pH value of 7.5 to 9.5 and at a temperature of 60 to 120°C.

## Revendications

1. Procédé de réduction de la teneur résiduelle en agent d'alkylation libre dans des solutions aqueuses d'agents tensioactifs contenant de l'azote, dans lequel on retraite des solutions d'agents tensioactifs cationiques, amphotères et/ou zwitterioniques avec de la monoéthanolamine à température accrue et le cas échéant à pression accrue.

2. Procédé selon la revendication 1,
caractérisé en ce qu'
on met en oeuvre en tant qu'agents tensioactifs cationiques, des esterquats de formule (I) 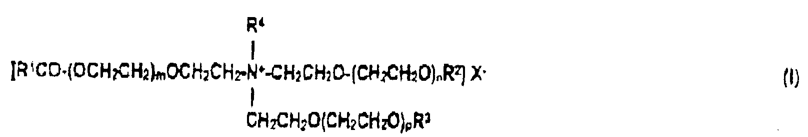 dans laquelle R¹CO représente un radical acyle ayant de 6 à à 22 atomes de carbone, R² et R³ indépendamment l'un de l'autre représentent de l'hydrogène ou R¹CO, R⁴ représente un radical alkyle ayant de 1 à 4 atomes de carbone ou un groupe (CH₂CH₂O)_{q}H-, m, n et p globalement représentent 0 ou des chiffres allant de 1 à 12, q représente un nombre allant de 1 à 12 et X représente un halogénure un alkylsulfate ou un alkylphosphate.

3. Procédé selon la revendication 1,
caractérisé en ce qu'
on met en oeuvre en tant qu'agents tensioactifs cationiques, des esterquats de formule II, 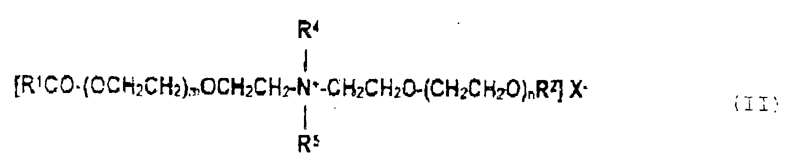 dans laquelle R¹CO représente un radical acyle ayant de 6 à 22 atomes de carbone, R² représente de l'hydrogène ou R¹CO et R⁴ et R⁵ indépendamment l'un de l'autre, représentent des radicaux alkyle ayant de 1 à 4 atomes de carbone, m et n globalement représentent 0 ou des nombres allant de 1 à 12, et X représente un halogénure, un alkylsulfate ou un alkylphosphate.

4. Procédé selon la revendication 1,
caractérisé en ce qu'
on met en oeuvre en tant qu'agent tensioactif cationique, des esterquats de formule III, 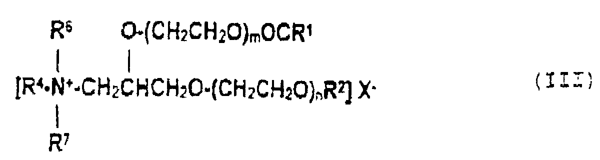 dans laquelle R¹CO représente un radical acyle ayant de 6 à 22 atomes de carbone, R² représente de l'hydrogène ou R¹CO, R⁴, R⁶ et R⁷ indépendamment l'un de l'autre représentent des radicaux alkyle ayant de 1 à 4 atomes de carbone, m et n globalement représentent 0 ou des nombres allant de 1 à 12, et X représente un halogénure un alkylsulfate ou un alkylphosphate.

5. Procédé selon la revendication 1,
caractérisé en ce qu'
on met en oeuvre en tant qu'agents tensioactifs cationiques des composés d'ammonium quaternaires de formule IV 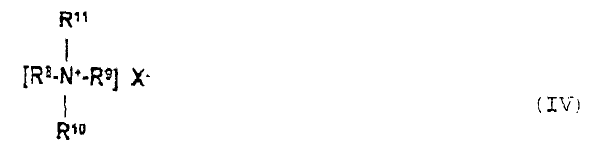 dans laquelle R⁸ et R⁹ indépendamment l'un de l'autre, représentent des radicaux alkyle éventuellement substitués par un hydroxy, ayant de 6 à 22 atomes de carbone, R¹⁰ représente des radicaux alkyle éventuellement substitués par un hydroxy ayant de 1 à 22 atomes de carbone, R¹¹ représente des radicaux alkyle ayant de 1 à 4 atomes de carbone et X représente un halogénure, un alkylsulfate ou un alkylphosphate.

6. Procédé selon la revendication 1,
caractérisé en ce qu'
on met en oeuvre en tant qu'agents tensioactifs amphotères ou zwitterioniques des alkylbetaïnes de formule (V), 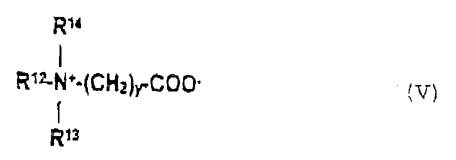 dans laquelle R¹² et R¹³ indépendamment l'un de l'autre représentent des radicaux alkyle éventuellement substitués par un hydroxy ayant de 6 à 22 atomes de carbone, R¹⁴ représente de l'hydrogène ou un radical méthyle et y représente 1,2 ou 3.

7. Procédé selon la revendication 1,
caractérise en ce qu'
on met en oeuvre en tant qu'agents tensioactifs amphotères ou en tant qu'agents tensioactifs zwitterioniques des alkylamidobétaïnes de formule VI, 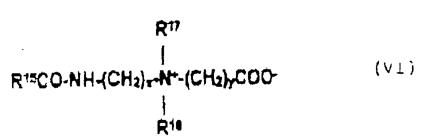 dans laquelle R¹⁵CO représente un reste acyle aliphatique ayant de 6 à 22 atomes de carbone, R¹⁶ représente un radical alkyle éventuellement substitué par un hydroxy, ayant de 1 à 22 atomes de carbone, R¹⁷ représente un hydrogène, ou un radical méthyle et x et y indépendamment l'un de l'autre représentent 1, 2 ou 3.

8. Procédé selon les revendications 1 à 7,
caractérisé en ce qu'
on retraite les solutions avec de 0,1 à 10 % en poids rapporté à la teneur en matières solides, de monoéthanolamine.

9. Procédé selon les revendications 1 à 8,
caractérisé en ce qu'
on retraite les solutions pendant un laps de temps de i à 4 heures, à une valeur de pH de 7,5 à 9,5 et à une température de 60 à 120°C.
